# EUROPEAN PATENT APPLICATION

(11) **EP 2 316 405 A1**
(43) Date of publication of application: **04.05.2011**
(21) Application number: 09425438.0
(22) Date of filing: 02.11.2009
(51) Int. Cl.: A61H 19/00

(54) **Apparatus for stimulation of tissues, particularly for female erogenous zones**

(71) Applicant: Tecnologica S.A.S. di Travaglini Cinzia & C., 61032 Fano (PS) (IT)
(72) Inventor: Vanella, Simone, 61122 Pesaro (IT)
(74) Representative: Modiano, Micaela Nadia

(57) **Abstract**

An apparatus (1) for the stimulation of tissues, particularly for female erogenous zones, which comprises a hollow box-like body (2) adapted to accommodate at least one suitable electric power supply unit for at least one laser radiation source and a respective control and management unit for adjusting the duration of the emission of the laser beam and of the working emission frequency of the laser beam emitted through a window (4) of the box-like body (2). The apparatus (1) further comprises a test assembly, which indicates the correct emission of the laser radiation.

## Description

The present invention relates to an apparatus for stimulation of tissues, particularly for female erogenous zones.

It is known to use sexual stimulators which, by way of a mechanical action that simulates sexual intercourse, can lead a woman to orgasm or to particular states of excitement.

If reduced sensitivity to sexual stimulation occurs, with reduced capacity for the woman to reach orgasm, it is often necessary to intervene pharmaceutically or surgically: this choice, however, is not shared by many women, due to the embarrassment linked to the request for such a therapy or surgical procedure.

It is also noted that as the age of a woman rises, some problems linked to the sexual context may also worsen, making it difficult and rare to have intense, long and repeated orgasms.

Recently, in order to increase clitoral sensitivity, medicine has proposed some types of surgical procedure, which consist in injecting an amount of hyaluronic acid on the clitoris.

The effectiveness of these treatments is ensured for a period of no more than 12 months, after which, if one wishes to extend the effect further, it is necessary to repeat the surgical procedure.

This procedure requires dealing with all the problems of a surgical procedure, albeit a small one, such as admission to day hospital, convalescence, anesthesia, et cetera.

Moreover, aging causes an aesthetic and functional deterioration of the tissues of the erogenous zones.

In order to improve the aesthetics of the female sexual organ, physicians propose a surgical procedure that consists in injecting a small quantity of silicone in sagging labia majora and/or labia minora.

The surgical procedure is to be repeated when the silicone is absorbed fully or partly by the body.

In this case also, since this is a surgical procedure, all the drawbacks mentioned earlier must be considered.

The effectiveness of this procedure also has a limited duration over time, since silicone tends to be absorbed by the body and after a certain period it is necessary to repeat the procedure, in a manner similar to what occurs for procedures for increasing lip volume.

It is necessary to add to all of the above the possible discomfort of a woman in going to a physician with such particular requests and in undergoing surgical procedures on her genitalia.

The aim of the present invention is to provide an apparatus for the stimulation of tissues, particularly for female erogenous zones, which allows the user, following a period of repeated treatments, to have orgasms if she does not have them normally, or to have more intense, longer and repeated orgasms and, as a consequence of a period of repeated treatments, to have an increased clitoral sensitivity.

Within this aim, an object of the present invention is to provide an apparatus for the stimulation of tissues, particularly for female erogenous zones, that can perform a toning, reinvigorating and rejuvenating action on the treated tissues.

Another object of the present invention is to provide an apparatus for the stimulation of tissues, particularly for female erogenous zones, that can slow the natural process of aging of the treated tissues.

Another object of the present invention is to provide an apparatus for the stimulation of tissues, particularly for female erogenous zones, that is capable of performing an action of functional improvement of the treated tissues.

A further object of the present invention is to provide an apparatus for the stimulation of tissues, particularly for female erogenous zones, which has a low cost, is relatively simple to provide in practice and safe in application.

This aim and these objects, as well as others which will become better apparent hereinafter, are achieved by the present apparatus for the stimulation of tissues, particularly for female erogenous zones, characterized in that it comprises a hollow box-like body adapted to accommodate one or more electric power supply means for at least one laser source and respective control and management means for adjusting the duration of the emission of the laser beam and of the working emission frequency of the laser beam emitted through a window of said box-like body.

Further characteristics and advantages of the invention will become better apparent and evident from the following detailed description of a preferred but not exclusive embodiment of an apparatus for the stimulation of tissues, particularly for female erogenous zones, illustrated by way of non-limiting example in the accompanying drawings, wherein:

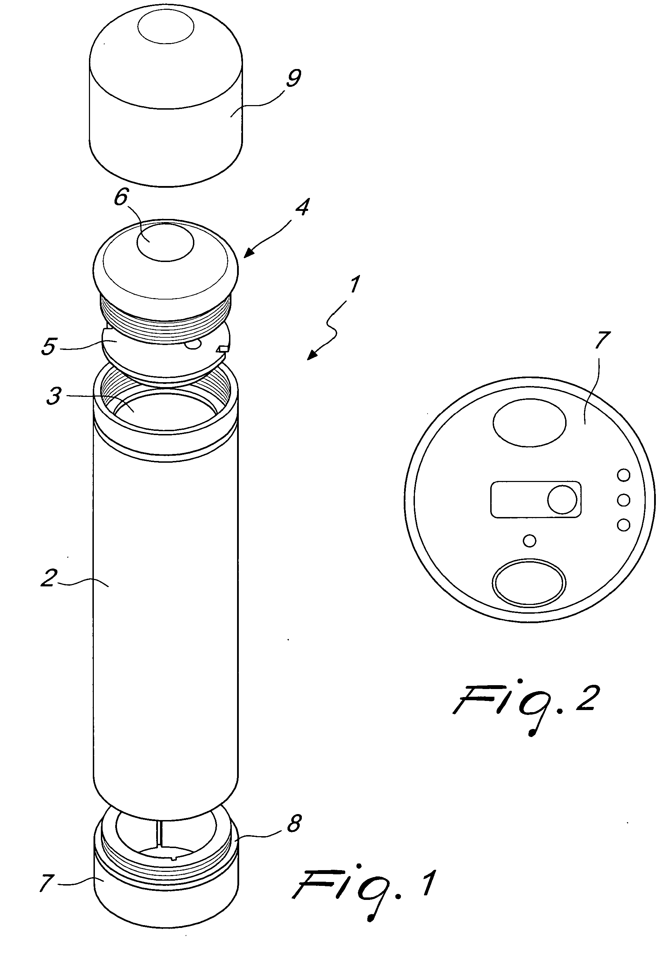

Figure 1 is an exploded perspective view of an apparatus for the stimulation of tissues, particularly for female erogenous zones, according to the invention;
Figure 2 is a plan view of the control panel of the apparatus for the stimulation of tissues, particularly for female erogenous zones, according to the invention.

With reference to the figures, the reference numeral 1 generally designates an apparatus for the stimulation of tissues, particularly for female erogenous zones.

The apparatus 1 for the stimulation of tissues comprises a box-like body 2 which has an internal cavity 3.

The cavity 3 is adapted to accommodate one or more electric power supply means for at least one laser source (it is specified immediately that although the embodiment that has a single source can be considered to be particularly effective, the adoption is not excluded of two or more sources whose operation may be synchronous, combined and/or independent) and respective control and management means for adjusting the duration of the emission of the laser beam and of the working emission frequency of the laser beam emitted through a window 4 of the box-like body 2.

In particular, the at least one laser source is accommodated on a respective plate 5, so that its position is determined uniquely within the box-like body 2 in order to utilize a beam emitted at the correct focal distance from the surface of the tissue to be treated.

The laser beam is, for example, at wavelengths comprised between 500 and 1000 nm.

The frequency of the emission pulses of the laser diode corresponds to values comprised between 500 and 10,000 Hz.

The times of the treatments are comprised by way of example between 5 and 25 minutes.

The control and management means (not visible in the figure) define the operating setting of the laser source, defining the emission frequency and the duration of the emission time of the laser beam in order to obtain the effect of stimulating and sensitizing the affected tissues.

The emission of the laser beam corresponds to a high-frequency beam of the emission pulses (a frequency which by way of example corresponds to wavelengths comprised between 500 and 10,000 Hz) with a sensitizing effect on the tissues that it strikes.

The high-frequency setting is particularly recommended for the treatment of female erogenous zones (the main objective of the present invention) when cases occur in which sexual pleasure is reached rarely (or is never reached) or one wishes to achieve more intense, longer and repeated orgasms and therefore suitable erogenous stimulation is required. Sensitizing the tissues in fact entails greater receptiveness to external stimuli on the part of the tissues and allows to overcome this type of problem, achieving the desired result.

By choosing preferred frequencies within the high-frequency operating range and the emission times of the laser beam, it is possible to direct the treatment toward increasing the sensitivity of tissues (to facilitate the achievement of orgasm to women), to rejuvenating/enhancing the tone of said tissues, and to slowing the aging of the tissues and a functional improvement of the female sexual organ.

By means of a suitable selection of the frequencies and duration times of the laser beam emission, these treatments can be performed independently of each other or, if it is possible to deliver a high-frequency light beam that comprises all of the spectrum that corresponds to the range between 400 and 800 Hz (or any subset thereof), within a single treatment.

According to the embodiment shown in the figures, the box-like body 2 is substantially cylindrical.

The possibility is not excluded from the outset of providing apparatuses that have an elliptical, oval and/or polygonal cross-section with angles which are suitably radiused to avoid damage of the tissues that come into contact with them.

The box-like body 2 comprises suitable end plugs 6 and 7, which can be accommodated detachably and hermetically at the ends of the box-like body 2.

The plugs 6 and 7 are provided with respective sealing gaskets (only the toroidal gasket 8 is visible in the figure).

The plug 6 is substantially at least partially translucent and transparent to the laser beam emitted by the source: the translucent portion of the plug 6 therefore constitutes the window 4 through which the laser beam can reach the surface of the tissue to be treated.

The transparent window 4, which constitutes the central part of the plug 6, can be a lens for concentrating or diffusing the beam emitted by one or more laser diodes, and this will enhance and amplify the effects of the treatment being performed.

The plug 7 comprises at least one element for control and adjustment of the functions for the user; in particular, the plug 7 on the bottom is provided with an actual control panel, by means of which it is possible to switch on and off the apparatus 1, report visually which type of treatment is in progress, and select such treatment.

It should be specified that the apparatus 1 can comprise (according to an embodiment of particular interest in practice, which in no way limits the possibility to have an apparatus 1 without this accessory) a suitable covering dome 9, which can be fitted on the top of the apparatus 1, substantially above the plug 6: the dome 9 acts as a protection for the plug 6.

The possibility is not excluded of providing a dome 9 that behaves instead like an optical filter, being able, according to requirements, to diffuse the emitted light beam, concentrate it or filter it in frequency.

The dome 9 is shaped like a hood whose shape is complementary to the shape of the upper surface of the apparatus 1 and also covers completely the plug 6.

According to an embodiment of particular interest in practice and in application, the translucent plug 6 is made of polymethyl methacrylate, known by the commercial name Plexiglass®: the qualities of this material from a mechanical standpoint (excellent strength and rigidity), from the production standpoint (it can be manufactured by molding and can be subjected to subsequent mechanical working after extraction from the mold) and from an optical standpoint (optimum transparency to radiations of specific interest), make it one of the most suitable for this type of application. However, the possibility is not excluded to use glass and/or other translucent polymeric materials to provide the plug 6.

The plug 6 is preferably provided with the shape and optical properties of a lens with suitable focal characteristics: the concentration and/or distribution emitted by the laser source can in fact constitute an important advantage of the apparatus 1. More specifically, with reference to a possible constructive solution, it is noted that the laser beam is emitted by the respective diode with a solid aperture angle of 15°: after it has passed through the lens (plug 6), the resulting laser beam propagates toward the surface to be treated in a manner that is parallel to the longitudinal axis of the apparatus 1.

It should be noted that according to a particular embodiment, the electric power supply means comprise at least one respective battery of accumulators: such battery is accommodated within the box-like body 2. The use of batteries (preferably of the rechargeable type, so that the apparatus can be charged with a suitable power supply connected to the electrical mains) has the advantage of eliminating completely the risk of electric shock linked to contact with live parts in the case of direct supply of power from the electrical mains.

In any case, if an apparatus 1 that is directly powered by the electrical mains is provided, suitable power supplies are adopted which comprise a voltage transformer which keeps the voltage values in the apparatus 1 below a predefined limit threshold, in order to avoid any danger or inconvenience for the user even in case of fault.

The laser source is preferably a laser diode which produces a laser beam whose wavelength is comprised substantially between 500 and 1000 nm: the possibility is not excluded, however, to adopt sources of a type other than laser diodes and/or to use beams emitted with wavelengths that differ even significantly from the reported values (typical value of laser diodes used in the medical and cosmetic fields).

The possibility is not excluded to pair laser diodes (LEDs) also with other light sources, in order to combine the effects of the beam emitted by the LED with the one or ones emitted by another source or sources, amplifying and enhancing the effects on the surface to be treated.

A laser diode is a laser source in which the active component is a semiconductor similar to the ones used in the production of LEDs (Light Emitting Diode). The most practical and common type of laser diode is formed by a p-n junction supplied by injected electric current. These devices are often referenced as injection laser diodes to distinguish them from optically pumped ones, which are easier to manufacture in the laboratory. The purpose is to release a considerable amount of energy in short times, allowing to treat very effectively, for example, most inflammatory states. The technology for the manufacture of laser diodes is based substantially on the emission of photons of monochrome light, polarized starting from the junction of two semiconductors, within which electrons are injected as a consequence of an adequate voltage. There is further a system of lenses which conveys the electron beam, collimating it at the output on optical fiber or directly externally.

The use of the apparatus 1 for the stimulation of tissues, particularly for the treatment of (generally) female erogenous regions and specifically in case of stimulation and sensitizing of tissues, consists in juxtaposing the window 4 on an area of the surface of tissue to be treated and activating the emission of a laser beam with a low wavelength of the emission pulses (by way of indication, between 500 and 900 nm).

It is then necessary to maintain the contact of the window 4 with the surface to be treated, by moving the apparatus 1 along the entire surface or only in a confined region (clitoris).

Conveniently, the apparatus 1 for stimulation of tissues, particularly for female erogenous zones according to the invention, allows the user to achieve orgasm more easily and, following a period of repeated treatments, to achieve better predisposition to achieving orgasms and to having more intense, longer and repeated orgasms.

Advantageously, the apparatus 1 is capable of performing a toning, reinvigorating and rejuvenating action on the treated tissues.

During aging, the external genitalia lose their turgidness due to reduction of elasticity and hydration of the tissues owing to degeneration and hypoproduction of elastin and collagen fiber due to fibroblastic hypoactivity and basophilic degeneration of the elastic fibers.

The pinkish color becomes less intense due to reduced surface vascularization and the vaginal tunic becomes increasingly relaxed due to thinning of the mucosal tunic, with reduced maturation of pavement epithelium and hypotrophy of the striated mucosa.

Substantially, the progressive shriveling and collapse of tissues occurs exactly as it occurs in all other body areas (face, breast, et cetera).

In order to contrast what has been noted above, one particularly effective method is the use of infrared laser aimed at stimulating and improving microcirculation by increasing the inflow of nutrients, tissue oxygenation, electrolytes, enzymes and vitamin factors and by increasing the production of type I collagen, for rejuvenation of the tissues and to delay their natural aging process.

Accordingly, today, by means of the apparatus 1 it is possible to achieve excellent results in a manner that is absolutely non-traumatic on the female sexual organ and also perform a preventive action that consists in slowing the natural aging process, both from an aesthetic standpoint and from a functional standpoint, maintaining adequate lubrication.

Positively, the apparatus 1 is capable of slowing the natural aging process of the treated tissues.

It is not secondary to note that the apparatus 1 for stimulating tissues, particularly for female erogenous zones, is capable of performing an action of functional improvement of the treated tissues.

It has thus been shown that the invention achieves the proposed aim and objects.

The invention thus conceived is susceptible of numerous modifications and variations, all of which are within the scope of the appended claims.

The possibility is specified to provide the apparatus with a plurality of laser sources, preferably conveyed into a single lens or each facing a suitable transparent area, so as to perform treatment on a plurality of areas of the surface of the skin that faces such openings.

The laser source or sources can be arranged not only at the tip but also in the lateral part of the cylindrical box-like body 2.

This solution can prove to be interesting if a cylindrical shape is adopted, but can also be applied with different configurations which can be traced back to substantially regular geometric solids or to irregular shapes or shapes which reproduce anatomical parts and/or fancy shapes.

The possibility is not excluded to adopt the inventive concept of the apparatus 1 according to the invention on mechanical stimulators for sexual use of the known type in order to produce a synergistic effect of mechanical stimulation with the stimulation produced by the light beam.

All the details may further be replaced with other technically equivalent ones.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the shapes and dimensions, may be any according to requirements without thereby abandoning the protective scope of the appended claims that follow.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An apparatus for the stimulation of tissues, particularly for female erogenous zones, **characterized in that** it comprises a hollow box-like body (2) adapted to accommodate one or more electric power supply means for at least one source of laser radiation and respective control and management means for adjusting the duration of the emission of the laser beam and of the working emission frequency of the laser beam emitted through a window (4) of said box-like body (2).

2. The apparatus according to claim 1, **characterized in that** said control and management means define settings for the operation of said laser source which correspond, by way of indication, to wavelengths comprised between 500 and 1000 nm and with frequencies comprised between 500 and 10,000 Hz and with emission times comprised between 5 and 25 minutes.

3. The apparatus according to claim 1, **characterized in that** said box-like body (2) has a shape that is chosen among a substantially cylindrical shape, a shape that reproduces an anatomical part, and an irregular fancy one.

4. The apparatus according to claim 3, **characterized in that** said box-like body (2) comprises end plugs (6, 7), which can be accommodated hermetically and detachably at the ends of said box-like body (2), said plugs (6, 7) being provided with respective sealing gaskets (8).

5. The apparatus according to claim 3, **characterized in that** at least one of said plugs (6) is substantially transparent to the laser beam emitted by said source and is provided with the shape and optical properties of a lens that has focal characteristics adapted to collimate the emitted laser beam.

6. The apparatus according to claim 3, **characterized in that** said plug (7) comprises at least one element for control and adjustment of the functions for the user.

7. The apparatus according to one or more of the preceding claims, **characterized in that** it comprises a covering dome (9), which can be fitted on the top of the apparatus (1), substantially over said plug (6).

8. The apparatus according to one or more of the preceding claims, **characterized in that** said electric power supply means comprise at least one respective battery of accumulators.

9. The apparatus according to one or more of the preceding claims, **characterized in that** said laser source is a laser diode that produces a laser beam whose wavelength is comprised substantially between 500 and 1000 nm.

10. Use of the apparatus (1) for stimulation of tissues, according to one or more of the preceding claims, particularly for erogenous zones and specifically in case of stimulation and sensitizing of tissues, comprising the steps that consist in juxtaposing said window (4) with respect to an area of the surface of tissue to be treated; activating the emission of a laser beam with high frequency of the emission pulses, maintaining the contact of the window (4) with the small surface of the clitoris or moving the apparatus (1) along the entire surface to be treated of the female genital organ.

11. A mechanical stimulator, particularly for female erogenous zones, **characterized in that** it comprises an apparatus according to one or more of claims 1 to 9.
